# EUROPÄISCHE PATENTSCHRIFT

(11) **EP 1 329 449 B1**
(45) Veröffentlichungstag und Bekanntmachung des Hinweises auf die Patenterteilung: **20.10.2004**
(21) Anmeldenummer: 02027094.8
(22) Anmeldetag: 04.12.2002
(51) Int. Cl.: C07D 307/89, B01D 3/14, C07C 51/573

(54) **Kolonne zur Aufkonzentration von Phtalsäureanhydrid**
Column for the concentration of phtalic anhydride
Colonne de concentration de l'anhydride phtalique

(30) Priorität: 22.01.2002 DE 10207460
(43) Veröffentlichungstag der Anmeldung: 23.07.2003
(73) Patentinhaber: Lurgi AG, 60295 Frankfurt am Main (DE)
(72) Erfinder: Gutermuth, Thomas, 63477 Maintal (DE); Domes, Helmuth, 63179 Obertshausen (DE)

(56) Entgegenhaltungen:
- EP-A- 0 087 678
- EP-A- 1 233 012
- DE-A- 4 336 986
- DE-A- 19 939 629
- FR-A- 1 261 584
- US-A- 3 725 211

## Beschreibung

Die Erfindung betrifft eine Destillationskolonne zur Herstellung von Phthalsäureanhydrid (PSA).
Roh-PSA enthält eine Reihe von leichter- und schwerersiedenden Nebenprodukten wie z.B. Benzoesäure, Maleinsäureanhydrid, Phthalid etc., die aus Gründen der Anforderungen an die Qualität des Rein-PSA weitestgehend entfernt werden müssen.
Bisher wird Roh-PSA in einer ersten Destillationsstufe von leichtersiedenden Nebenprodukten befreit. In einer zweiten Destillationsstufe werden schwersiedende Komponenten abgetrennt und Rein-PSA wird als Kopfprodukt gewonnen.
Die Destillation des Roh-PSA wurde bislang wie folgt durchgeführt: Das von der thermischen Vorbehandlung kommende Roh-PSA (dort wird Wasser entfernt, bzw. Phthalsäure zu Phthalsäureanhydrid umgewandelt) gelangt durch gravimetrische Höhenunterschiede in die sogenannte Vorbehandlungskolonne. Dort wird das Roh-PSA zwischen dem obersten Boden und der darüberliegenden strukturierten Packung in flüssiger Form eingeleitet und in die Kolonne entspannt. Im Sumpf der Vorbehandlungskolonne wird das Roh-PSA verdampft. Die aufsteigenden Dämpfe stehen im Stoffaustausch mit der herabrieselnden Flüssigkeit aus dem Zulauf des Roh-PSA bzw. des Kopfkondensators. Aus dem Kopfkondensat, in dem die leichtersiedenden Nebenprodukte (zusammen mit PSA) aufkonzentriert sind, wird ein geringer Teil abgezogen und zum sogenannten Rückstands-Behälter als Abfall gefahren. Der Sumpf in der Vorbehandlungskolonne ist damit von Leichtsiedern befreit. Die siedende Flüssigkeit im Sumpf wird gravimetrisch in den Sumpf der Rein-PSA-Kolonne gefördert. Dies geschieht mit Hilf eines Überlaufs. Diese Kolonne arbeitet als reine Verstärkungskolonne und ist mit 20 Ventilböden ausgestattet. Am Kopf der Rein-PSA-Kolonne wird das Rein-PSA abgenommen und zum Teil als Rücklauf auf den oberen Boden gegeben. Der andere Teil wird als Reinprodukt entnommen. Der Sumpfrückstand der Kolonne (Schwersieder) wird aus der Kolonne abgezogen und in einem Aufkochgefäß diskontinuierlich auf ca. 250 ° C aufgeheizt, um darin enthaltenes PSA durch Verdampfung zu gewinnen. Diese apparative Ausführung der Destillation hat sich über Jahrzehnte hinaus bewährt, hat aber den Nachteil, dass zwei komplette Kolonnen erforderlich sind. Dies erfordert nicht nur hohe Investitionskosten, sondern auch den nötigen Platzbedarf für die dazugehörige Stahlkonstruktion. Beide Kolonnen arbeiten im Unterdruckbereich bei ca. 140 mbar und verfügen über getrennte Vakuumsysteme.

Kolonnen für die Aufkonzentration von Phthalsäureanhydrid sind unter anderem in der DE-A-3538911 beschrieben. Hier werden zwei nebeneinander angeordneten Destillationskolonnen zur Herstellung von z.B. Phthalsäureanhydrid verwendet. Die EP-B-0087678 beschreibt eine sehr hoch bauende Bodenkolonne, ebenfalls zur Reinigung von Phthalsäureanhydrid.
Bekannt sind ebenfalls Kolonnen, die zur Vermeidung der Quervermischung von Flüssigkeits- oder Dämpfeströmen mittels Trennwand in Teilbereichen der Kolonne geteilt werden. Dies wird in der DE-A-3522234 beschrieben. In der DE-A-4336986 ist eine Kolonne beschrieben, die über den gesamten Querschnitt mittels Trennblech geteilt ist. In dieser Kolonne ist in beiden Kolonnenhälften je ein Stoffaustauschpaket angeordnet.
Beide Kolonnen sind für die Herstellung von Phthalsäureanhydrid nicht geeignet.

Ausgehend von diesem Stand der Technik liegt der Erfindung die Aufgabe zugrunde, die bisherige Kolonnenanordnung apparativ zu verbessern und kostengünstiger sowie montagefreundlicher zu gestalten.

Erfindungsgemäß wird die Aufgabe bei der eingangs genannten Kolonne dadurch gelöst, dass in der Kolonne zur Aufkonzentration von Phthalsäureanhydrid zwei Destillationsstufen angeordnet sind, wobei die Abdestillation der Leichtsieder im Roh-PSA in der ersten Destillationsstufe erfolgt und die Abtrennung der Schwersieder aus dem Rein-PSA in der zweiten Destillationsstufe durchgeführt wird, wobei beide Destillationsstufen nebeneinander angeordnet sind und durch eine senkrecht angeordnete Wand voneinander getrennt sind und der Sumpf der ersten Destillationsstufe mit dem Sumpf der zweiten Destillationsstufe verbunden ist

In der nachfolgenden Ausführung wird die erste Destillationsstufe als Vorkolonne bezeichnet und die zweite Destillationsstufe als PSA-Kolonne.
Da der Sumpfablauf der Vorkolonne und der Sumpfzulauf der PSA-Kolonne auch bei der Ausführung mit zwei getrennten Kolonnen die gleiche Zusammensetzung und beide Sümpfe praktisch die gleichen Drücke und Temperaturen haben, kann der Vorkolonnen-Sumpf mit Hilfe eines Überlaufrohres direkt in den Sumpf der PSA-Kolonne weitergeleitet werden. Damit entfällt die bisherige gravimetrische Förderung des Sumpfes aus der Vorkolonne in den Sumpf der PSA-Kolonne.

Da die Vorkolonne und die Rein-PSA-Kolonne mit unterschiedlichen Aufkochraten betrieben werden, müssen beide Stufen der Trennblechkolonne mit je einem Aufkocher und einem Kopfkondensator ausgerüstet sein.
Die Kondensatoren werden über einen gemeinsamen Wasserzu- und Wasserablauf gespeist.

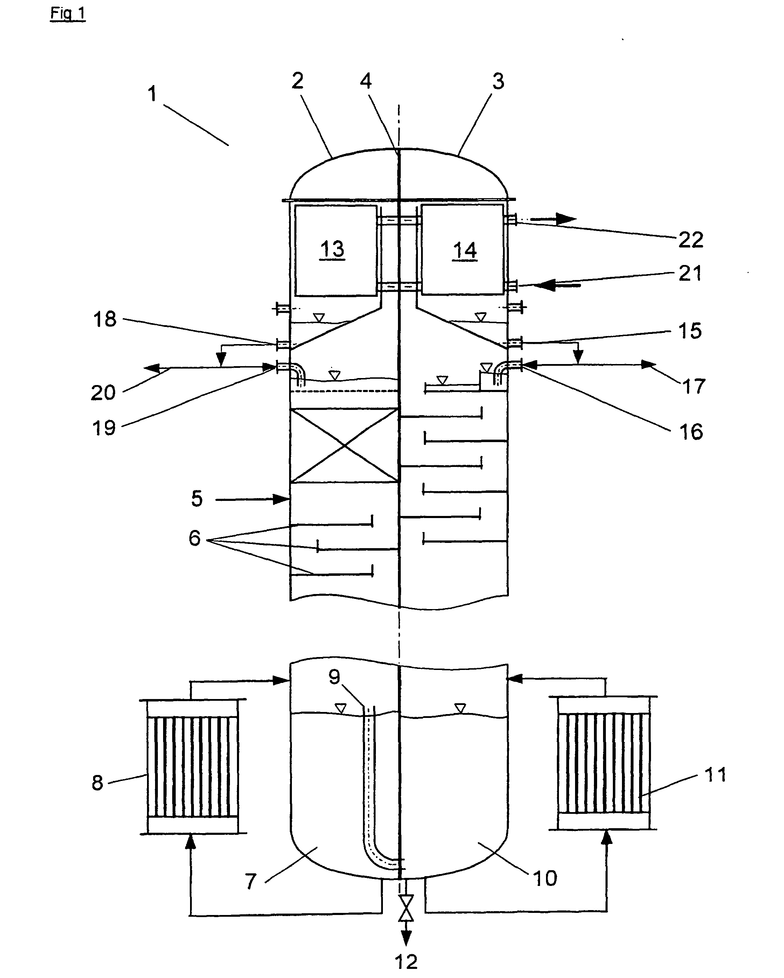

Ausgestaltungsmöglichkeiten der Kolonne werden mit Hilfe der Zeichnung beispielhaft erläutert.

Bei der erfindungsgemäßen Modifikation der Destillation von Roh-PSA kommt anstelle der beiden getrennten Kolonnen eine sogenannte Trennwandkolonne (1) zum Einsatz, die verfahrenstechnisch die gleiche Aufgabe erfüllt wie die beiden separaten Kolonnen. Der zylindrische Kolonnenkörper der Trennwandkolonne (1) wird auf der ganzen Länge inklusive der Böden sowie im Sumpf und Kopf mit Hilfe einer Wand (4) in zwei halbkreisförmige Segmente unterteilt. Die Wand (4) kann aus Edelstahl-Blech oder anderen chemisch beständigen Werkstoffen bestehen, die die entstehende Belastung aufgrund der Temperatur und des Druckes aufnehmen können. Die Zeichnung zeigt auf der linken Seite die Vorkolonne (2) und auf der rechten Seite die Rein-PSA-Kolonne (3). In der Vorkolonne (2) erfolgt die Destillation des Roh-PSA (erste Destillationsstufe) und in der Rein-PSA-Kolonne (3) die Destillation des Rein-PSA (zweite Destillationsstufe).
Das von der thermischen Vorbehandlung kommende flüssige Roh-PSA (5) wird in die Vorkolonne (2) entspannt. Die entstehenden Dämpfe steigen in der Vorkolonne (7) auf, während der flüssige Anteil des entspannten PSA über die Ventilböden (6) in den Sumpf (7) der Vorkolonne (2) rieselt. Das Aufkochen des Sumpfes (7) erfolgt über einen Umlaufkocher (8). Da der Sumpfablauf der Vorkolonne (2) und der Sumpfzulauf der Rein-PSA-Kolonne (3) auch bei der Ausführung mit zwei getrennten Kolonnen die gleiche Zusammensetzung und beide Sümpfe praktisch die gleichen Drücke und Temperaturen haben, kann der Vorkolonnen-Sumpf (7) mit Hilfe eines Überlaufrohres (9) direkt in den Sumpf der PSA-Kolonne (10) weitergeleitet werden. Alternativ ist die Förderung der Flüssigkeit mittels Pumpe möglich.

Das PSA im Sumpf (10) der PSA-Kolonne (3) wird mit Hilfe eines Umlaufkochers (11) aufgekocht und am Kopf der PSA-Kolonne (3) als Rein-PSA durch einen Kondensator (14) zurückkondensiert, wobei ein Teil als Rücklauf über Leitung (15) und Leitung (16) in die PSA-Kolonne (3) zurückläuft und der andere Teil als Reinprodukt (17) gewonnen wird. Im Kolonnensumpf (10) sammeln sich Schwersieder, die kontinuierlich in das Aufkochgefäß über Leitung (12) abgeleitet werden.

Im Kopf der Vorlaufkolonne (2) werden mit Hilfe des Kondensators (13) Leichtsieder rückkondensiert, die am Kolonnenkopf über Leitung (18) und Leitung (19) teils als Rückfluss wieder in die Vorkolonne (2) eingeleitet werden und zu einem geringen Teil über Leitung (20) als Rückstand gesammelt werden.
Im Kopf der Vorkolonne (2) und im Kopf der PSA-Kolonne (3) sind je ein Kondensator (12, 14) angeordnet, die über einen gemeinsamen Zu- (21) und Ablauf (22) mit Speisewasser versorgt werden; die produktseitig anfallende Kondensationswärme wird zur Dampferzeugung genutzt.
Der Einsatz einer Trennwand (4) ist für diese Destillationsanwendung möglich, da auf beiden Seiten der Trennwandkolonne (1) fast gleiche Temperatur- und Druckverhältnisse herrschen:

| Vorlaufkolonne | | |
|---|---|---|
| Temperatur | Sumpf: 228 ° C | Kopf 200 ° C |
| Druck | Sumpf 0,24 bar abs | Kopf: 0,14 bar abs |

| PSA-Kolonne: | | |
|---|---|---|
| Temperatur | Sumpf: 228 ° C | Kopf 211 ° C |
| Druck | Sumpf 0,24 bar abs | Kopf: 0,14 bar abs |

Damit sind nur geringe mechanische Belastungen auf die Trennwand infolge von Druck- und Temperaturdifferenzen zu erwarten.

Um die Trennblechkolonne unter Vakuumbedingungen betreiben zu können, sind am Kopf der Kolonne Absaugstutzen (nicht dargestellt) für die nicht kondensierbaren Dämpfe vorgesehen. Dabei ist jede Kolonnenseite mit einem separaten Stutzen ausgerüstet. Für beide Kolonnen kommt ein gemeinsames Vakuumsystem (nicht dargestellt) zum Einsatz.

## Patentansprüche

1. Kolonne zur Aufkonzentration von Phthalsäureanhydrid mit zwei Destillationsstufen, in der die Abdestillation der Leichtsieder im Roh-PSA in der ersten Destillationsstufe (2) erfolgt und die Abtrennung der Schwersieder aus dem Rein-PSA in der zweiten Destillationsstufe (3) durchgeführt wird, wobei beide Destillationsstufen nebeneinander angeordnet sind und durch eine senkrecht angeordnete Wand (4) vollständig voneinander getrennt sind, **dadurch gekennzeichnet,**
**dass** der Sumpf (7) der ersten Destillationsstufe (2) mit dem Sumpf (10) der zweiten Destillationsstufe (3) verbunden ist.

2. Kolonne nach Anspruch 1, **dadurch gekennzeichnet, dass** der Sumpf (7) der ersten Destillationsstufe (2) durch ein Überlaufrohr (9) mit dem Sumpf (10) der zweiten Destillationsstufe (3) verbunden ist.

3. Kolonne nach Anspruch 1, **dadurch gekennzeichnet, dass** der Sumpf (7) der ersten Destillationsstufe (2) durch eine Pumpe mit dem Sumpf (10) der zweiten Destillationsstufe (3) verbunden ist.

4. Kolonne nach Anspruch 1, **dadurch gekennzeichnet, dass** beide Kolonnenteile mit einem gemeinsamen Vakuumsystem betrieben werden.

5. Kolonne nach Anspruch 1, **dadurch gekennzeichnet, dass** die Trennwand (4) aus Blech besteht.

6. Kolonne nach Anspruch 1, **dadurch gekennzeichnet, dass** zur ersten Destillationsstufe (2) und zur zweiten Destillationsstufe (3) je ein Umlaufkocher (8, 11) zugeordnet ist.

7. Kolonne nach Anspruch 1, **dadurch gekennzeichnet, dass** zur ersten Destillationsstufe (2) und zur zweiten Destillationsstufe (3) je ein Kondensator (13, 14) zugeordnet ist.

## Claims

1. A column for concentrating phthalic anhydride with two distillation stages, in which the removal of the low-boiling components in the crude phthalic anhydride by distillation is effected in the first distillation stage (2) and the separation of the high-boiling components from the pure phthalic anhydride is performed in the second distillation stage (3), both distillation stages being arranged side by side and being completely separated from each other by a vertical wall (4), **characterized in that** the bottom (7) of the first distillation stage (2) is connected with the bottom (10) of the second distillation stage (3).

2. The column as claimed in claim 1, **characterized in that** the bottom (7) of the first distillation stage (2) is connected with the bottom (10) of the second distillation stage (3) by an overflow pipe (9).

3. The column as claimed in claim 1, **characterized in that** the bottom (7) of the first distillation stage (2) is connected with the bottom (10) of the second distillation stage (3) by a pump.

4. The column as claimed in claim 1, **characterized in that** both column parts are operated with a common vacuum system.

5. The column as claimed in claim 1, **characterized in that** the partition (4) is made of sheet metal.

6. The column as claimed in claim 1, **characterized in that** to the first distillation stage (2) and to the second distillation stage (3) there is each associated a circulation boiler (8, 11).

7. The column as claimed in claim 1, **characterized in that** to the first distillation stage (2) and to the second distillation stage (3) there is each associated a condenser (13, 14).

## Revendications

1. Colonne de concentration d'anhydride phtalique, comprenant deux étages de distillation, dans laquelle la séparation par distillation des produits à bas points d'ébullition du PSA brut s'effectue dans le premier étage (2) de distillation et la séparation des produits à hauts points d'ébullition du PSA pur s'effectue dans le deuxième étage (3) de distillation, les deux étages de distillation étant disposés l'un à côté de l'autre et étant séparés entièrement l'un de l'autre par une paroi (4) verticale, **caractérisé,**
**en ce que** le pied (7) du premier étage (2) de distillation communique avec le pied (10) du deuxième étage (3) de distillation.

2. Colonne suivant la revendication 1, **caractérisé en ce que** le pied (7) du premier étage de distillation communique avec le pied (10) du deuxième étage (3) de distillation par un tube (9) de trop plein.

3. Colonne suivant la revendication 1, **caractérisé en ce que** le pied (7) du premier étage (2) de distillation communique avec le pied (10) du deuxième étage (3) de distillation par une pompe.

4. Colonne suivant la revendication 1, **caractérisé en ce que** deux parties de colonne fonctionnent avec un système sous vide commun.

5. Colonne suivant la revendication 1, **caractérisé en ce que** la paroi (4) de séparation est en tôle.

6. Colonne suivant la revendication 1, **caractérisé en ce qu'**il est associé au premier étage (2) de distillation et le deuxième étage (3) de distillation respectivement un bouilleur (8, 11) de recirculation.

7. Colonne suivant la revendication 1, **caractérisé en ce qu'**il est associé au premier étage (2) de distillation et au deuxième étage (3) de distillation un condenseur (13, 14).
